# EUROPEAN PATENT SPECIFICATION

(11) **EP 2 046 409 B1**
(45) Date of publication and mention of the grant of the patent: **10.04.2019**
(21) Application number: 07796930.1
(22) Date of filing: 19.07.2007
(51) Int. Cl.: A61L 31/08, A61B 42/00, A61L 31/14, A61L 31/10

(54) **NONAQUEOUS COATING COMPOSITION FOR ELASTOMERIC ARTICLES AND ARTICLES CONTAINING THE SAME**
NICHTWÄSSRIGE BESCHICHTUNGSZUSAMMENSETZUNG FÜR ELASTOMERE ARTIKEL UND DIESE ENTHALTENDE ARTIKEL
COMPOSITION DE REVÊTEMENT NON AQUEUSE POUR ARTICLES ÉLASTOMÈRES ET ARTICLES LES CONTENANT

(30) Priority: 20.07.2006 US 490458
(43) Date of publication of application: 15.04.2009
(60) Divisional application: 16192898.1
(73) Proprietor: Allegiance Corporation, McGaw Park, IL 60085-6787 (US)
(72) Inventor: WANG, Shiping, Libertyville, IL 60048 (US); CHEN, Seon Fong, Gelugor Penang, 11700 (MY); LOW, Chii Yhi, Penang, 11800 (MY); WONG, Wei Cheong, Kedah (MY); BERGER, Ida, Buffalo Grove, IL 60089 (US)
(74) Representative: Prock, Thomas
(86) International application number: PCT/US2007/016343
(87) International publication number: WO 2008/011088

(56) References cited:
- WO-A-02/092046
- WO-A-2004/037305
- US-A1- 2003 059 489
- US-A1- 2004 115 250
- US-A1- 2004 122 382
- US-A1- 2005 042 248
- US-B1- 6 274 154
- DATABASE WPI Week 200336 Derwent Publications Ltd., London, GB; AN 2003-376302 XP002474457 & JP 2003 027083 A (OKAMOTO CO LTD) 29 January 2003 (2003-01-29)
- DATABASE WPI Week 199519 Derwent Publications Ltd., London, GB; AN 1995-143910 XP002474458 & JP 07 067906 A (SAGAMI RUBBER KOGYO KK) 14 March 1995 (1995-03-14)
- DATABASE WPI Week 198841 Derwent Publications Ltd., London, GB; AN 1988-289758 XP002474459 & JP 63 212355 A (KATSUSHIMA A) 5 September 1988 (1988-09-05)

## Description

### BACKGROUND OF THE INVENTION

The invention relates to the field of medical devices. In particular, the invention relates to a nonaqueous coating composition for skin-contacting surfaces of elastomeric articles. Elastomeric articles, which are used in contact with the wearer's skin, are well known. Articles such as medical gloves and condoms, for example, can be worn by the user for extended periods of time. Because certain elastomeric articles are used with relatively higher frequency as well as with prolonged duration, important characteristics of such articles include their physical properties and their comfort of use.

A variety of medical gloves, e.g., surgical gloves and examination gloves, are well known and readily available in the medical field. The chemical and physical properties of elastomers used in such gloves have been researched, and gloves exhibiting desirable properties in accordance with their usage have been developed. Properties such as tensile strength and elongation modulus, as well as coatings and lubricants, which enhance their usage and/or donning characteristics, have been investigated. A variety of elastomeric polymer compositions have been examined as well, including formulations using natural and synthetic latex.

When gloves are worn for extended periods of time, body heat is generated by the hand and perspiration that can cause overhydration damaging the protection afforded by the stratum corneum. After the gloves are removed from the hand and the sweat evaporates, the skin of the hand can become dry, sensitive and sometimes, infected. Such undesirable skin conditions can lead to even more serious skin problems as a result of the loss of epidermal lipid barrier layer, which preserves skin moisture.

Pre-donning skin lotions have been developed for application to the user's skin prior to donning gloves. Such lotions are typically applied separately to the skin, and the glove is then donned afterward. Other lotions are applied to the skin after the glove has been removed. Therapeutic skin-moisturizing gloves containing water-activatable material on a skin-contacting surface are described in Berry U.S. Patent No. 5,869,072. The water-activatable material disclosed in this reference includes polyvinyl alcohol, as well as additional ingredients such as moisturizers. Chou U.S. Patent No. 6,274,154 describes an elastomeric glove wherein the skin-contacting surface contains an aloe vera coating in the dry state. One problem associated with many lotions or creams is the deterioration of glove performance as a result of adverse effects on barrier and physical properties of the elastomer. Another problem associated with pre-coated gloves is their ability to withstand sterilization treatment and/or elevated thermal environments, encountered during the manufacturing process and storage. Yet another problem with such lotions or creams is the use of oily emollients, which can produce an uncomfortable greasy feeling.

Certain elastomeric articles, such as surgical gloves, are worn for extended periods of time during medical procedures. The comfort, maintenance of skin moisture, and reduction of skin irritation have become of increasing interest. One difficulty associated with developing elastomeric gloves which are both functional and comfortable to the user's skin has been the balancing of their desirable physical (e.g., tactile) attributes in combination with beneficial and therapeutic results for the user's skin. Even more difficult is the accomplishment of these physical and comfort characteristics while at the same time also providing thermal stability and topical therapeutic benefit.

WO 2004/037305 A1 discloses a therapeutic, moisturizing coating composition for elastomeric articles which is applied directly onto the skin-contacting surface of the article as part of the manufacturing process. The coating composition is thermally stable and subsequently hydrates when contacted with a moisturized skin surface to convert into a liquid "lotion" form during wearing of the article.

US 6 274 154 B1 discloses a moisturizing and therapeutic glove which includes a thin layer of Aloe Vera coated evenly and uniformly on an inside surface of the glove.

US 2004/115250 A1 discloses a glove wherein the interior surfaces of the glove contain a film which includes water, glycerol and a botanical extract.

US 2004/122382 A1 discloses an elastomeric article, such as an elastomeric glove, having a coating on the skin-contacting surface of the article that includes a carrier and a clinically beneficial additive.

US 2003/059489 A1 discloses a genital lubricant composition comprising i) fatty acids and/or homeopathic dilutions of plant extracts and ii) a physiologically acceptable carrier. According to a preferred embodiment, the genital lubricant composition comprises about 0.05% to about 0.5% hemp seed oil and a physiologically acceptable carrier so that it forms a lotion, a cream or a gel.

US 2005/042248 A1 discloses a substantially anhydrous warming, non-toxic and non-irritating lubricating composition containing polyhydric alcohols and an insulating agent as well as gel and jelly compositions related thereto.

JP 2003-027083 A discloses an aqueous lubricant containing a tea polyphenol having a virus-inhibiting amount of the tea polyphenol. Coloring of underwear is avoided by setting the caffeine content of the tea polyphenol within a definite range.

JP H07-67906 A discloses a lubricant composition for a condom comprising glycerol and polyvinyl pyrrolidone to provide a lubricant composition for a condom having satisfactory lubricity

JP S63-212355 A discloses a moisture-holding and lubricating viscous-membrane protecting agent for condoms.

WO 02/092046 A1 discloses a skin care composition comprising by weight: (1) from about 0.01% to about 5% of a carboxylic acid/carboxylate copolymer; (2) from about 0.1% to about 2% of a surfactant system comprising two or more nonionic surfactants selected from the group consisting of polyoxyalkylene alkyl ethers having a C12-18 alkyl substitute, polyoxyalkylene hydrogenated castor oils, and linear or branched, mono- or trialkyl glycerides; (3) from about 0.05% to about 5% of a silicone component; (4) from about 0.01% to about 5% of an emollient oil; (5) from about 0.1% to about 10% of a sebum absorbing agent; (6) from about 1% to about 20% of a water soluble humectant; and (7) an aqueous carrier; wherein the weight ratio of the surfactant system to the emollient oil is from about 2:1 to about 1:1; and wherein a base composition consisting essentially of components (1) through (4), (6) and (7) above has an absorbance of no more than about 2 at a wavelength of 340 nm.

Accordingly, there is a need in the field of skin-contacting elastomeric articles for improvements in their comfort to the user. Particularly advantageous would be the development of an elastomeric glove which is pre-coated with a nonaqueous therapeutic skin care treating composition which is thermally stable.

### SUMMARY OF THE INVENTION

The invention provides an elastomeric article comprising a therapeutic coating composition on the skin-contacting surface that can be applied to the article during its manufacture and subsequently afford a comfortable and therapeutic effect to the wearer's skin while maintaining the desirable physical properties of the article. A coating composition has been discovered which is compatible with medical gloves, is thermally stable, has no substantial adverse effect on its physical properties, has reduced irritation of the wearer's skin, has a non-sticky feel, has good surface-to-skin transference, and has reduced inter-surface and intra-surface tackiness between like elastomeric articles. Particularly surprising is it has been discovered that not only does the coating composition reduce the adverse effects of wearing elastomeric articles over time, but it improves the condition of the wearer's skin. Even more surprising is that such a formulation could be developed with a combination of ingredients that can "survive" the conditions of article (e.g., glove) manufacturing. Elastomeric articles with which the invention can be used include industrial gloves, medical gloves (i.e., examination and surgical gloves), condoms, and the like. The invention is particularly useful in examination and surgical gloves. The invention provides a nonaqueous therapeutic coating applied to the skin-contacting surface of the elastomeric article, said coating composition comprising a moisturizing agent; and wherein said coating composition is applied directly onto said elastomeric article surface. The therapeutic nonaqueous coating composition transfers onto the wearer's skin during use, providing the topical benefits afforded by the ingredients of the composition.

According to an aspect of the invention there is provided a nonaqueous therapeutic coating composition for the skin-contacting surface of an elastomeric article, said coating composition comprising:
glycerin in an amount ranging from 50.00% to 90.00% by weight of the composition; and
sorbitol in an amount ranging from 0.1% to 6.0% by weight of the composition;
wherein said composition is essentially water-free and transfers upon contact with skin; and,
wherein the nonaqueous therapeutic coating composition is adapted for application directly onto the skin-contacting surface of the elastomeric article.

According to another aspect of the invention there is provided a nonaqueous therapeutic coating composition for the skin-contacting surface of an elastomeric article, said coating composition comprising:
glycerin in an amount ranging from 50.00% to 90.00% by weight of the composition;
sorbitol in an amount ranging from 0.1% to 6.0% by weight of the composition; and
a transferable film-forming polymer, an exfoliant, or microporous particles;
wherein said composition is essentially water-free and transferable upon contact with skin; and,
wherein the nonaqueous therapeutic coating composition is adapted for application directly onto the skin-contacting surface of the elastomeric article.

According to another aspect of the invention there is provided an elastomeric article comprising a therapeutic coating composition of the invention as described above on the skin-contacting surface of said article.

According to another aspect of the invention there is provided a process for the production of an elastomeric article, said process comprising contacting the skin contacting surface of said elastomeric article with a nonaqueous therapeutic coating composition of the invention as described above.

The nonaqueous coating composition continues to provide prolonged therapeutic benefit to the skin following its removal. The coating composition is chemically compatible with the elastomeric materials, and has no substantial impact on the physical properties of the article. The coating composition of the invention is thermally stable and survives elevated temperatures associated with manufacturing and certain sterilization treatments. Additional ingredients can be combined with the composition of the invention, such as lubricants, anti-tacking agents, antimicrobial agents and time release or sustained release agents as well.

An important aspect of the coating composition of the invention is the collective skin moisturization efficacy of multiple coating composition ingredients. The therapeutic skin properties of the coating composition are accomplished in part by the discovery that some of the ingredients possess dual functionalities, wherein at least one of their functions is beneficial skin moisturization. The moisturizing functionality of the coating composition is premised upon at least two of the following moisturization effects. First, some of the ingredients of the composition function as water-soluble (or perspiration-soluble) moisturizers, such as glycerin and sorbitol. Second, some of the ingredients function as skin penetrative moisturizers, such as panthenol. Third, some ingredients can function as prolonged skin surface moisturizers, such as film-forming polymers such as chitosan. Furthermore, combinations of the above can be employed to suit the nature of wear associated with different elastomeric article types. For example, since examination gloves are worn for relatively shorter time periods, the prolonged skin surface moisturizer(s) used can be optional.

Also disclosed herein is a nonaqueous coating composition for skin-contacting surfaces of elastomeric articles comprising at least one polyhydric alcohol moisturizer and at least one alphahydroxy lactone. Preferred polyhydric alcohol moisturizers are glycerol, sorbitol, and pantothenol. A preferred alphahydroxy lactone is gluconolactone. Also disclosed herein is an elastomeric article comprising a nonaqueous coating composition on the skin-contacting surface, the coating comprising at least one polyhydric alcohol moisturizer and at least one alphahydroxy lactone.

The term "transfer" is meant to refer to the means by which the nonaqueous coating composition of this invention is transmitted to the wearer's skin. After donning the glove, the coating is transferred to the wearer's skin. The transfer or transmission of the coating to the skin is facilitated by the perspiration and body temperature of the wearer. Also, the action of the glove rubbing against the skin will also assist with the transference. Also disclosed herein is a process for making a skin-contacting elastomeric article providing enhanced therapeutic properties to the skin of the wearer, the process comprising : applying a nonaqueous coating composition to the skin-contacting surface of the elastomeric article, the coating composition comprising at least one polyhydric alcohol moisturizer and at least one alphahydroxy lactone. The process can be used in the manufacture of examination and surgical gloves. Also disclosed herein is a method of therapeutically treating the skin on the hands of an individual in need of said treatment comprising the steps of: providing an elastomeric glove comprising a nonaqueous coating composition comprising at least one polyhydric alcohol moisturizer and at least one alphahydroxy lactone; donning the glove on the hand; wearing the glove for a period of time sufficient to permit transfer of the coating composition onto the skin surface of the hand; and subsequently removing the glove from the hand. The therapeutic treatments include improved skin moisturization, reduced flaking, softness of feel, improved skin elasticity and firmness, reduced redness and irritation, and reduced appearance of wrinkles. Additional embodiments and advantages of the invention will become apparent from the following description.

### DETAILED DESCRIPTION OF THE INVENTION

The term "therapeutic" is meant to refer to the effect of improving skin-related properties of moisture, elasticity, comfort, non-irritation, preservation of protective skin barrier properties, and the like.

The term is used relative to "dry state" or "nonaqueous state", which is used herein to indicate the substantial absence of moisture or water.

As used herein, the term "thermally stable" and "thermal stability" when referring to the properties of the coating composition of the invention is meant to indicate that the coating composition can withstand elevated temperatures of about 90° C. The nonaqueous coating composition according to the invention is adapted for application directly onto a skin-contacting surface of an elastomeric article as part of the manufacturing process. The processing temperature includes both the temperature at which the formulation is prepared and the temperature at which the formulation is applied to the glove. The processing temperature for coating composition may range from about 20°C to about 100°C. The heating decreases the viscosity of the formulation and assists with the ease of application to the skin-contacting side of the glove.

The coating composition of the invention is particularly suited for elastomeric articles which include, as part of their anticipated usage, intimate contact with a wearer's skin surface and prolonged residence thereon. Suitable skin-contacting elastomeric articles include, but are not limited to, gloves (e.g., industrial, medical, and surgical gloves), condoms, finger cots, and the like. Elastomeric articles per se to be treated according to the invention can be manufactured using conventional techniques and equipment readily available to those skilled in the art. For example, elastomeric gloves can be made using conventional former-dipping-curing techniques and equipment, such as that described in Yeh, U.S. Patent No. 6,391,409.

Elastomers or elastomeric substrates upon which the coating composition can be applied can include any natural or synthetic elastomeric polymer which is chemically compatible with the coating composition ingredients and appropriate for the intended use, e.g., surgical environment. Suitable elastomers include, but are not limited to, synthetic and natural rubber latex. Natural rubber that can be used includes rubber made from *hevea* rubber latex and *guayule* rubber latex. Synthetic rubber polymers which can be used include nitrile rubber, polyurethane, polyisoprene, polychloroprene, styrene block co-polymers and blends thereof. Synthetic rubbers that can be used also include acrylic diene block co-polymers, acrylic rubber, butyl rubber, EPDM rubber, polybutadiene, chlorosulfonated polyethylene rubber, and fluororubber.

One important characteristic of the invention is that the nonaqueous coating composition can be applied directly onto the surface of the article as part of the manufacturing process. The coating composition is packaged, stored, and presented in a solid state on the article surface. Thus, when in intimate contact with the skin surface the coating composition converts into a liquid "lotion" form during wear. It is during this stage that the coating composition transfers and provides an initial therapeutic and moisturizing benefit to the wearer which remains on the skin surface for a period of time after the article is removed.

Glycerin has a dual functionality within the nonaqueous coating composition of this invention. It serves as both a carrier and a moisturizer. The term "carrier" is meant to refer to an organic, stable, non-volatile material, which serves as a vehicle for delivering ingredients of the nonaqueous coating composition on to the glove. It is a liquid at either or both room temperature and at the processing temperature. It has a boiling point above 100°C and a vapor pressure ranging from about 0.001 mm Hg to about 760 mm Hg at 25°C.The "carrier" in this nonaqueous composition is considered a unique property in this invention.

Other examples of organic carriers include: polyhydric alcohols such as polyglycerols such as diglycerol and polyglycerol-3; glycols such as ethylene glycol, propylene glycol, butylene glycol, diethylene glycol, dipropylene glycol, triethylene glycol and tripropylene glycol; alkoxylated alcohols: such as glycereth-7 and glycereth-26; fatty acid esters such as ethylene glycol esters such as glycol stearate, glycol palmitate and glycol oleate; propylene glycol esters such as propylene glycol myristate and propylene glycol laurate; fatty acid mono- and diglycerides such as glyceryl laurate, glyceryl oleate, diglycerin caprirate and diglycerin oleate; ethoxylated triglycerides such as polyethylene glycol caprylic/capric triglycerides, polyethylene glycol caprylic/capric glycerides,; ethoxylated glycerol esters such as polyethylene glycol glyceryl fatty esters; polyoxyethylene diglycerol ethers such as POE (6) diglycerol ether and POE (40) diglycerol ether; polyoxypropylene diglycerol ethers such as POP (4) diglycerol ether and POP (24) diglycerol ether; and polypropylene glycol ethers such as PPG-14 butyl ether and PPG-3 myristyl ether. The carrier within can also consist of a combination of any of the materials listed above.

Moisturizers that can be used in the coating composition include polyhydric alcohol emollients and/or moisturizers. At least one moisturizer is present, but combinations of two or more moisturizers can be used as well. Suitable polyhydric alcohol moisturizers that can be used include, but are not limited to, glycerin and sorbitol. A combination of glycerin and sorbitol is used. An example of glycerin or 1,2,3-propanetriol that can be used is Glycon™ G 300 (available from Aldrich Chemical Company, Milwaukee, Wisconsin). One example of sorbitol or D-glucitol that can be used is available from Aldrich Chemical Company, Milwaukee, Wisconsin.

Moisturizer ingredients can be present in an amount up to about 95.00% by weight of the total composition. Preferably, the moisturizer is present in an amount ranging from about 10.00% to about 95.00% by weight. Glycerin moisturizer is present individually in an amount ranging from about 50.00% to about 90.00% by weight, preferably from about 60.00% to about 89.00% by weight of the total nonaqueous composition. Sorbitol as a moisturizer is present individually in an amount ranging from about 0.1% to about 6.00%, preferably from about 0.50% to about 4.00% by weight.

Additional moisturizing agents can be used in conjunction with the above-described polyhydric alcohols. The additional moisturizing agents can likewise be polyhydric alcohols. Preferably, the composition of the invention comprises
pantothenol or 2,4-dihydroxy-N-(3-hydroxypropyl)-3,3-dimethylbutanamide (a.k.a. pro-vitamin B), which, in vivo, increases topical moisture retention thereby prolonging elasticity and suppleness of the skin, reduces inflammation and irritation of the skin, and stimulates epithelization. Suitable pantothenol for use in the invention is available from Aldrich Chemical Company, Milwaukee, Wisconsin and Daiichi Fine Chemicals, Japan, as well as Ritapan™ DL (available from RITA Corporation, Woodstock, Illinois).

The pro-vitamin B form, pantothenol, is preferred for use in the composition. Pantothenoic acid, the acid form of pantothenol, is a member of the B-complex vitamins and is also a structural component of acyl carrier proteins (ACP) as an essential component of fatty acid synthetase complex. The stability of pantothenoic acid is, however, highly sensitive to pH fluctuations. The pro-vitamin form pantothenol is preferred because it is more stable and easily absorbed, and converts into the acid form in vivo. Additional moisturizing agents such as pantothenol can be present in an amount ranging from about 0.10% to about 5.00% by weight, preferably from about 1.50% to about 3.00% by weight of the total nonaqueous composition.

The coating nonaqueous composition can further comprise a hydroxyacid as a skin exfoliant. Hydroxyacids enhance proliferation of skin cells and increases ceramide biosynthesis keratinocytes, regulates epidermal thickness and improves desquamation resulting in smoother skin and youthful appearance. Suitable hydroxyacids that can be used include monocarboxylic acids, dicarboxilic acids and polyhydroxy acids, and their intramolecular lactones, esters and salt forms. Examples of monocarboxilic acids include both alpha and beta forms. Gluconolactone or D-glucono-1,5-lactone is preferred because it provides a therapeutic effect with relatively less skin irritation. One example of gluconolactone that can be used is Glucono-delta-Lactone available from Daniels Archer Midland/DL, United Kingdom or Jungbunzlauer, Newton Center, Massachusetts. Hydroxyacid(s) such as gluconolactone can be present in an amount ranging from about 0.10% to about 5.00% by weight, preferably from about 0.10% by weight to about 2.00% by weight of the total nonaqueous composition.

Donning agents that can be used include quaternary ammonium halide salts which additionally exhibit antimicrobial properties. A preferred quaternary ammonium halide salt is cetylpyridinium chloride or 1-hexadecylpyridinium chloride. One example of cetylpyridinium which can be used is CPC available from Zeeland Chemicals, Zeeland, Michigan.

Other donning agents that can be used include silicone-based compounds, including polyalkylsiloxanes. One example of a polyalkylsiloxane that can be used is polydimethylsiloxane dispersion,. Other silicone-related donning agents can be used that also function as skin protecting agents as well, such as dimethicone.

Donning agents such as cetylpyridinium chloride can be present in an amount ranging from about 0.00% to about 8.00% by weight, preferably from about 0.10% to about 6.00% by weight of the total nonaqueous composition. Other donning agents can be present in an amount ranging from about 0.10% to about 8.00%, preferably from about 0.10% to about 0.25% by weight of the total composition. The coating composition can also include a pH adjuster, which can be an inorganic acid, organic acid, or combination thereof. The amount of pH adjuster to be added will, of course, vary but is preferred that the compound and amount be selected to adjust the pH value to from about pH 4 to about pH 7. Preferred are non-irritating pH adjusters, such as citric acid or 2-hydroxy-1,2,3-propane-tricarboxylic acid, which is available from Aldrich Chemical Company, Milwaukee, Wisconsin. The pH adjuster(s) such as citric acid can be present in an amount ranging from about 0% to about 2.00% by weight of the total nonaqueous composition.

Anti-tack agents that can be used in the coating composition include silicones such as silicone oil, silicone resins, silicone gums and silicone elastomers, cationic polymers such as polydiethyldimethyl ammonium chloride, fatty acid salts and esters such as potassium stearate and trimethylolpropane triisostearate, carboxylic ester of hydroxyalkylamide such as erucamide, fluoro-compounds such as PTFE (polytetrafluoroehtylene) and phosphate salts such as ammonium alkyl phosphate (such as Darvan L™ available from R.T. Vanderbilt, Norwalk, Connecticut). Anti-tack agent(s) can be present in an amount ranging from about 0.25% to about 8.00% by weight of the total nonaqueous composition.

The nonaqueous coating composition of the invention preferably contains a hydration promoter to facilitate uptake and absorption of topical moisture (water) needed to "activate" the coating composition. Preferred hydration promoters include those, which additionally function as buffers to an acidic pH adjuster when present, such as sodium citrate. One example is 1,2,3-propanetricarboxylic acid trisodium salt, such as sodium citrate dihydrate available from Aldrich Chemical Company, Milwaukee, Wisconsin. Hydration promoter(s) such as sodium citrate can be present in an amount ranging from about 0% to about 8.00%, preferably from about 0.5% to about 4.00% by weight of the total nonaqueous composition.

Other therapeutic and cosmetic agents can be used as well, such as "anti-aging" compounds. Cosmetic agents that can be used include retinol, and/or those that can also function as exfoliants, such as alpha hydroxy lactones, such as gluconolactone. Fragrances and coloring agents can also be used in the nonaqueous coating formulation to render the composition more appealing to the user.

As a further embodiment, the coating composition can comprise a plasticizer to facilitate uniform distribution. Preferred plasticizers include esters such as triethyl citrates, because of its additional chemical function as a buffer in the formulation. One example of suitable triethyl citrate or 1,2,3-propanetricarboxylic acid, 2-hydroxy-, triethyl ester is Hydagen™ CAT available from Cognis, Cincinnati, Ohio. Plasticizer(s) such as Hydagen™ CAT can be present in an amount ranging from 0% to about 1.00%, preferably from 0% to about 0.50% by weight of the total composition.

Prior to application to the surface of the elastomeric article, the inventive nonaqueous coating composition needs to be heated, so as to be applied to the surface in liquid state. The nonaqueous coating composition can be heated to temperatures from about 20°C to at least about 100°C prior to glove application. It can be applied using a variety of coating techniques. Suitable coating techniques include dipping and spraying. The coating composition is subsequently cooled to a highly viscous or solid state as part of the manufacturing process. Application and finishing techniques that can be used in accordance with the invention include the tumbling process and the spraying process. The spraying method is preferred for preparing surgeon's or surgical gloves. The tumbling method is preferred for preparing examination gloves. The examples contain descriptions of each of these processes in greater detail.

The spray method is a process that applies the heated coating composition onto the glove using a spraying apparatus. In this process, the gloves are placed into a tumbler including a spraying device. The coating composition is sprayed in the liquid state in multiple and non-continuous steps. Alternatively, the tumbling method is a process that applies the coating composition in liquid state onto the glove surface by placing the gloves into a tumbler and then filling the tumbler with the coating composition liquid. The gloves are then tumbled or washed. A tumbling process that can be used, for example, can be similar to that described in Chen et al. U.S. patent application Serial No. 10/666,650 filed September 17, 2003, now pending. Additional processes are discussed in patent applications: 10/676,793 filed September 30, 2003, 10/719,573 filed November 22, 2003, and 11/056,628 filed February 9, 2005.

An important aspect of the coating composition of the invention is the collective skin moisturization efficacy of multiple coating composition ingredients, and some of the coating composition ingredients of the invention can possess dual functionality. The humectant functionality of the coating composition is premised upon at least two of the following moisturization effects. First, some of the ingredients of the composition function as moisturizers, such as glycerin and sorbitol. Second, some of the ingredients function as skin penetrative moisturizers, such as pantothenol. Third, some ingredients can function as prolonged skin surface moisturizers, such as film-forming polymers such as chitosan. Furthermore, combinations of the above can be employed to suit the characteristics associated with different elastomeric article types. For example, since examination gloves are worn for relatively shorter time periods, the prolonged skin surface moisturizer(s) used can be optional.

Variations of the above ingredients and the addition of other ingredients are possible in accordance with the invention, provided the therapeutic effect of the formulation on the skin is not significantly compromised. The ingredients of the coating composition, when combined, must be capable of participating in the therapeutic, moisturizing, non-irritating, moisturizing effect on the wearer's skin.

When the elastomeric article is intended for extended wear, such as a surgeon's glove, the coating composition preferably further comprises a water-soluble film forming polymer. Suitable water-soluble, film forming polymers which can be used include natural or synthetic film forming polymers. Preferred are cationic carrier -soluble film forming polymers. Suitable film forming polymers which can be used in accordance with the invention include, but are not limited to, cellulose and cellulose derivatives, polyvinyl pyrrolidone (PVP) and polyvinyl pyrrolidone derivatives, and polysaccharides. Preferably, polysaccharides are used as the carrier-soluble film-forming polymer, and most preferred, as the film-forming polymer is chitosan.

Chitosan can be prepared from naturally occurring chitin, which can be obtained from crustacean and insect exoskeletal material. Chitosan is also referred to as deacetylated chitin, poly-D-glucosamine, poliglusam, beta-1,4-poly-D-glucosamine, and beta-(1,4)-2-amino-2-deoxy-D-glucose. Chitosan and its derivatives can be used in accordance with the invention. One source of chitosan that can be used is deacetylchitin and its derivatives which are soluble/dispersible in the carrier of this invention. When present, film-forming polymer(s) such as chitosan can be present in an amount ranging from 0.00% to about 1.00% by weight of the total composition.

Water should not be present in any significant amount. Preferably, water is present up to about 1.0% by weight of the total inventive coating composition. Those skilled in the art may appreciate that the carriers and components of the formulation are not absolutely anhydrous. Overall, variations of the proportions and amounts of the ingredients can be adjusted provided such modifications do not substantially compromise the desired properties of the resulting formulation according to the invention.

In another embodiment, particle technology can also be used in conjunction with the coating composition to further enhance the collective benefits and properties of the invention. In particular, microporous particles can be included in the formulation to provide a number of additional properties, such as sustained release or time release of ingredients. Preferably, the microporous particles that can be used are those impregnated with skin care ingredients that can be incorporated into the inventive coating. Microporous particle technology that can be used as a component of the coating composition of the invention includes that which is described in U.S. Patent No. Reissue No. 33,429, U.S. Patent No. 4,873,091, U.S. Patent No. 4,690,825, U.S. Patent No. 5,028,435, U.S. Patent No. 5,035,890, U.S. Patent No. 5,968,543, U.S. Patent No. 5,955,109, U.S. Patent No. 5,073,365, U.S. Patent No. 5,135,740, U.S. Patent No. 5,145,675, U.S. Patent No. 5,145,685, U.S. Patent No. 5,156,843, U.S. Patent No. 5,316,774, U.S. Patent No. 5,458,890, U.S. Patent No. 5,840,293, U.S. Patent No. 5,871,722, and U.S. Patent No. 5,851,538. One particular microsponge particle of interest is Microsponge 5700 (available from Cardinal Health, Inc., Somerset, New Jersey), which can controllably release particle-absorbed dimethicone by diffusion. The coating composition of the invention can further contain additional beneficial ingredients provided such are chemically compatible with the composition of the invention and do not adversely affect the desired therapeutic properties of the composition. Additional ingredients that can be included in the coating composition include, but are not limited to, antimicrobial agents, anti-inflammatory agents, topical cleansing agents, anti-perspiration agents, organic solvents, and the like.

The coating composition of the invention can also be applied to elastomeric article surfaces using conventional equipment and techniques readily available to those skilled in the field of manufacturing elastomeric articles, including on-line and off-line techniques such as dipping, spraying, tumbling, and the like. For preparing a
coated surgeon's glove, the preferred method of application is off-line spraying. For the preparation of a coated exam glove, the preferred on-line method of application is dip coating, and the preferred off-line method is the tumbling method of coating.

Using a surgeon's glove as an example, the user removes the glove from a dispenser or package. Prior to donning the glove, the user typically scrubs their hands with surgical scrub solution followed by rinsing with water. After wiping their hands dry with a sterile towel, the user dons the glove by placing the hand into the glove such that the glove generally conforms to the shape of the user's hand. At this point, the moisture and heat from the user's skin interacts with the coating composition thereby hydrating and melting the composition and converting it into a liquid "lotion"-type phase. Upon hydration and melting of the coating composition, the comfort, and therapeutic benefits such as moisturization of the skin become realized. Furthermore, after removal of the glove, the coating composition remains on the user's skin thereby providing continued therapeutic benefit to the skin.

The following examples further illustrate the invention. Unless otherwise noted, % is meant to indicate percent by weight of the total weight of the nonaqueous composition.

### Example 1 - Previous Aqueous Preparation of Coating Composition for Surgical Gloves

A previous aqueous coating was prepared by initially determining the amount of each ingredient desired using conventional ingredient amount calculation methods. After the amount of ingredients has been determined, the total amount of water was added to a beaker and continually stirred as each of the ingredients were added. The composition was stirred at ambient temperature for at least about one hour until a stable, homogenous solution was formed. The pH and viscosity of the composition was measured in accordance with ASTM E70-97 (Standard Test Method for pH of Aqueous Solutions with the Glass Electrode) and ASTM D5225-98 (Standard Test Method for Measuring Solution Viscosity of Polymers with a Differential Viscometer). Using these methods, the pH was between 4.8 and 6.0, and the viscosity was measured at between 0.015 and 0.045 Pa.s (15 and 45 cps) at room temperature using a spindle no. 4 at 60 rpm. The resulting composition was then deposited into a glass container and sealed with a lid.

The resulting liquid coating formulation had the following composition:

**Formula 1:**

| **Ingredient:** | **Amount (w/w %)** |
|---|---|
| Chitosan | 0.10 |
| Citric acid | 0.10 |
| Glycerin | 0.25 |
| Sorbitol | 0.75 |
| Pantothenol | 0.50 |
| Gluconolactone | 0.25 |
| Triethyl citrate | 0.50 |
| Cetylpyridinium chloride | 1.00 |
| Silicone dispersion | 0.25 |
| Sodium citrate | 0.40 |
| Alkyl phosphate ammonium salt | 1.00 |
| Deionized water | 94.90 |
| **Total:** | 100.00 |

### Example 2A - Previous Aqueous Preparation of Coated Glove (Spraying Method)

A surgeon's glove containing a previous aqueous coating composition on the skin-contacting surface was prepared as follows:
The uncoated glove was prepared in accordance with conventional glove forming techniques and equipment. A former in the shape of a glove was provided and coated with a coagulant composition and subsequently dried. The coagulant-coated former was then dipped into polyisoprene latex to coat the former and the latex was leached with water, coated with a powder layer, and cured on the coated former. After curing, the polyisoprene glove was rinsed, dried, and stripped from the former.

Prior to coating, the gloves were turned inside-out and pre-rinsed and chlorinated using a chlorinator with chlorine solution with a chlorine strength of about 300 ppm to about 1000 ppm. After chlorination, the gloves were post-rinsed prior to coating.

To coat the glove, the chlorinated gloves were removed from the chlorinator, turned inside out such that the skin-contacting surface was exposed and placed in a tumbler equipped with a spray nozzle. The gloves were then sprayed. Then, the gloves were dried for a sufficient period of time.

Tumbler design is an important aspect of the preparation of the gloves in order to ensure an even uniform coating of the glove surface. For example, a drum having a diameter of about 43 inches and a total length of about 63.5 cm (25 inches) can have a total perforated area of about 29.2 cm (11.5 inches) and a remaining non-perforated area of about 34.3 cm (13.5 inches) relative to drum length. The non-perforated area of the drum is determined so that some of the gloves to be placed within remain in the non-perforated area for lubrication and so that suction air flow is reduced in the area. The revolution speed can vary. For example, a revolution speed ranging from about 25 rpm to about 35 rpm can be used, preferably from about 31 rpm to about 32 rpm.

Initial drying can be conducted at a temperature of about 32° C increasing to about 50° C, for a period of about 15 minutes. The first spraying can commence after 15 minutes of tumbling. After the first spraying, the gloves were then tumbled for an additional 60 seconds, and subjected to a second spraying for about 170 seconds and tumbled again for an additional 60 seconds. A third spraying was applied for about
170 seconds, followed by tumbling at a temperature of about 60° C for a period of about 2 minutes of cooling. The spraying step was repeated until the desired amount of coating has been applied to the gloves prior to 25 minutes heat tumbling and 2 minutes cooling cycle.

At the conclusion of the tumbling stage, the gloves were removed from the tumbler for the turning stage. During the turning process, the gloves were manually turned inside out and then subjected to the final drying at a temperature of about 55° C for a period of about 15 minutes. The gloves were then allowed to cool for a period of about 3 minutes. The gloves were then dried at a temperature of about 34° C for a period of about 20 minutes. The gloves containing the previous aqueous coating composition in dry-state can then be packaged and sterilized.

### Example 2B - Previous Aqueous Preparation of Coated Glove (Tumbling Method)

An examination glove containing a previous aqueous coating composition on the skin-contacting surface were prepared as follows:
Prior to coating, the gloves were post-processed by chlorination. First, the gloves were turned inside-out exposing the skin-contacting surface and placed into the chlorinator. The glove was then be pre-rinsed and chlorinated using a chlorinated solution with a chlorine strength of about 400 ppm to about 700 ppm. After chlorination, the gloves were post-rinsed prior to coating.

For coating, the chlorinated gloves were removed from the chlorinator and placed into a tumbler for aqueous coating and heat drying steps. Excessive water was removed from the gloves by spinning the gloves for a period of about 5 minutes. The tumbler was then filled with an aqueous lotion solution such as per Example 3, Formula 2 or Example 4, Formula 3. The gloves were then be tumbled in the composition for a period of about 10 minutes. The composition was then drained from the tumbler.

The gloves were dried in the tumbler in a heating cycle at a temperature of about 50° C for a period of about 30 minutes, and subsequently cooled in a cool-down cycle for a period of about 5 minutes. The gloves were then removed from the tumbler and manually turned inside-out. The gloves were then dried again in a dryer at a temperature of about 60° C for a period of about 60 minutes and then allowed to cool to room temperature for about 10 minutes.

In the preparation of coated articles according to the present invention used a process similar to Example 2B for examination gloves and Example 2A for surgeon's gloves except that the drying step was deleted. Cooling began after the application of the nonaqueous coating. One of ordinary skill in the art can make adjustments and modifications to the above process parameters as appropriate for particular circumstances.

### Example 3 - Previous Aqueous Lotion Formulation for Natural Rubber Examination Glove

**Formula 2:**

| **Ingredient** | **Amount (% w/w)** |
|---|---|
| Citric acid | 0.10 |
| Glycerin | 0.10 |
| Sorbitol | 0.30 |
| Pantothenol | 0.20 |
| Gluconolactone | 0.20 |
| Sodium citrate | 0.40 |
| Silicone dispersion | 0.12 |
| Alkyl phosphate ammonium salt | 0.74 |
| Water | 97.84 |
| **Total**: | 100.00 |

### Example 4 - Inventive Nonaqueous Coating

**Inventive Formula 3**

| **Ingredient** | **Amount (% w/w)** |
|---|---|
| Citric acid | 0.40 |
| Glycerin | 87.87 |
| Sorbitol | 3.03 |
| Pantothenol | 2.02 |
| Gluconolactone | 1.01 |
| Trisodium Citrate Dihydrate | 1.62 |
| Alkyl phosphate ammonium salt | 4.04 |
| Total: | 100.00 |

The inventive formula set out above was investigated against pure glycerin and deionized water for viscosity as measured against temperature. This test was conducted in accordance with ASTM D 2196-05. It is interesting to note that the glycerin based formula 3 had a consistently higher viscosity over the temperature range of 20-90°C. More specifically, at 20°C the inventive glycerin based formulation had a viscosity of about 2.2 Pa.s (2200 cP), while pure glycerin had a viscosity of about 1.75 Pa.s (1750 cP). This confirms that the inventive formulation is a highly viscous, almost solid composition at room temperature. One aspect of the present invention resides in the discovery that a nonaqueous lotion formulation that can be applied to the skin contacting surface of an elastomeric article. The inventive composition is applied by a novel nondehydration process wherein the lotion composition can be transferred to the skin during and after wearing the elastomeric article. The inventive nondehydration process and inventive composition provides the elastomeric article
with an enhanced performance without compromising glove properties such as barrier strength, donning ease, softness, and aging durability.

The inventive coating composition was prepared as follows. With continuous stirring at 50-65°C, the ingredients were added to glycerin as follows: pantothenol, gluconolactone, citric acid, trisodium citrate dihydrate, sorbitol, and Darvan L. There was a 10 minute lapse between the additions of each ingredient. The composition was stirred at 60°C increasing the temperature to about 90°C in about one hour. A stable homogeneous clear solution was formed. The pH was between 5.6 and 5.7 and the viscosity was measured at 16s (ASTM D 4212).

### Example 5 - Inventive Process.

This experiment was conducted to determine if there was any mass loss during the process of application. Reference is made to Examples 1 - 4. This example describes the treatment of both surgical and exam gloves.

Nontreated polyisoprene surgical gloves were prepared in accordance with conventional glove forming and equipment known to those skilled in the art. Prior to coating, the gloves were pre-rinsed and chlorinated with chlorine strength of about 300ppm to about 400ppm. After chlorination, the gloves were prerinsed and pretreated with lubricant prior to coating.

To treat the gloves, the gloves were turned inside out, such that the skin contacting surface is exposed. The gloves were then placed in tumbler equipped with a spray nozzle. The gloves were heated to about 70°C in the tumbler for about 5 minutes. The gloves were then sprayed with the inventive Formula 3, while spraying took place for a period of about 20 seconds. At the conclusion of spraying, the coating was allowed to solidify on the gloves with cooling at 60°C for about 30 minutes. The gloves were then cooled to ambient temperature in about 3 minutes and then inverted. The coating weight for the surgical glove was from about 50mg per glove to about 180mg per glove. The gloves were then packaged and sterilized. These treated, surgical polyisoprene glove will hereinafter be referred to as Sample 1.

Prior to the coating of the nitrile exam gloves, they were pre-rinsed with chlorine at a strength of about 600ppm to about 700ppm. After chlorination, the gloves were then pre-rinsed prior to coating. Nitrile exam gloves were treated in a similar manner as set out above for the surgical glove. The nitrile exam gloves were also coated using the formulation of the invention set out in Formula 3. It only differed in the solidification time of 35 minutes at 60°C. Thus, the nondehydration process can be applied to a variety of glove substrates. The coating weight of the exam gloves is from about 5mg per glove to about 80mg per glove.

### Example 6 - Inventive Nonaqueous Coating

**Inventive Formula 4:**

| **Ingredient** | **Amount (% w/w)** |
|---|---|
| Citric acid | 0.39 |
| Glycerin | 84.46 |
| Sorbitol | 2.91 |
| CPC | 3.89 |
| Pantothenol | 1.94 |
| Gluconolactone | 0.97 |
| Trisodium Citrate Dihydrate | 1.55 |
| Alkyl phosphate ammonium salt | 3.89 |
| **Total**: | 100.00 |

Sample 2 was prepared using Inventive Formula 4 and the inventive process of Example 5 (without the lubricant pre-treatment).

### Example 7 - Determination of Coating Weight

The coating weight of the gloves were determined by using an extraction process. First, ten treated gloves and ten non-treated gloves are placed in a desiccator for 30 minutes. Each set of gloves were weighed and inverted so that the skin contacting side would be washed. The gloves were washed and tumbled rigorously with water for about 2.5 minutes in a large container. The water was removed and the gloves were washed and tumbled three more times. The gloves were then dried for 30 minutes at 60°C. The gloves are then inverted and dried for an additional 30 minutes at 60°C. The gloves were then placed in a desiccator for 30 minutes and re-weighed.

The glove washing process for the exam glove differed from the surgical glove in three aspects. First, prior to being placed in the desiccator, the exam gloves were pre-dried at about 75°C for about 30 minutes. Second, the cuffs of the exam gloves were tied with rubber bands prior to glove washing. Third, the gloves were dried at 80°C for 30 minutes.

Various factors affect the appropriate amount (load level) of coating composition applied to the glove, such as glove temperature, composition temperature, number of sprays, distance of glove from spray nozzle, total solids content of composition. The amount of coating (lotion per glove) can be calculated from total lotion spray and load size (number of gloves). Sprayer settings also affect the coating process, including cylinder pressure, liquid pressure, air pressure, and air cap type. Adjustments to appropriate or optimal manufacturing parameters can be selected by those skilled in the glove manufacturing field.

### Example 8 - Inventive Glove Evaluation

### Glove Performance

### Section A: Treated vs. Non-Treated Glove

The glove performance was examined using a panel of evaluators, which involved nine individuals instructed to don and wear gloves treated with the inventive formulation and gloves that had received no treatment at all. Both the nontreated and treated (Sample 1) gloves were sterilized 34-40kGy and aged (at 40°C) for seven days, prior to the glove performance evaluation. Panelists donned the non-lotion treated and lotion-treated (Sample 1) gloves with dampened hands and wore them for 15 minutes. The evaluation required the individuals to assess the donning ability, transfer, quick dry, and silky feel properties of the glove on a scale of 1 through 4, 1 indicating a poor performance and a 4 indicating an excellent performance. Wet donning is the measure of the ease with which one can don the glove. Transfer is the amount of coating transferred to the hand after wearing the glove. Skin dry is the speed at which your hand dries after donning and removing the glove. The silky feel of the glove is defined as the smoothness and lack of tackiness after wearing the glove. The results for this study, utilizing a surgical glove, are shown in the following table:

| Glove ID | Wet Donning | Transfer | Skin Dry | Silky Feel |
|---|---|---|---|---|
| Non-Treated | 2.9 | 2.7 | 3.4 | 2.8 |
| Treated (Sample 1) | 3.1 | 3.0 | 2.5 | 2.4 |

The above evaluation demonstrates that a glove treated with the inventive non-aqueous formulation (Sample 1) provides a surprisingly better wet donning performance than the nontreated glove. Additionally, it gives a greater transfer of lotion to the skin. The skin drying at a slower rate further confirmed this affect. Overall, the silky feel of the glove is not negatively impacted by the greater transference of lotion to the hand.

### Section B: Non-aqueous Composition vs. Aqueous Composition

Surgical gloves, Sample 2, were prepared in a according to Example 5 above using Formula 3 (nonaqueous coating composition) and were compared with Sample 3, gloves treated with an aqueous coating composition, (Formula 4 of Example 5 of the parent patent). Both sets of gloves were sterilized 34-40kGy and aged (at 40°C) for seven days, prior to the glove performance evaluation. These gloves were evaluated according to the method described in Section A above. The results of this study are shown in the following table:

| Glove ID | Wet Donning | Transfer | Skin Dry | Silky Feel |
|---|---|---|---|---|
| Treated with non-aqueous composition (Sample 2) | 2.3 | 2.6 | 2.1 | 2.2 |
| Treated with aqueous composition (Sample 3) | 2.3 | 2.4 | 2.6 | 2.5 |

The above evaluation demonstrates that a glove treated with the non-aqueous coating composition of the present invention (Sample 2, Formula 4) provides a similar glove performance to a glove treated with an aqueous coating composition (Sample 3, see Formula 1). The therapeutic non-aqueous coating composition transfers onto the wearer's skin during use, providing the topical benefits such as good wet donning, transference, quick skin dry and silky feel, which is afforded by the ingredients of the composition, which mainly consists of the carrier glycerin.

### Physical Properties:

The physical properties of the coating on the glove's physical performance was evaluated. ASTM D 3577-01a was used for surgical gloves and ASTM D 6319-00 was used for the exam gloves. These were before and after values subsequent to aging at 70°C for 24 hours. The treated gloves were compared to the gloves without treatment. The evaluation of tensile strength, modulous at 300% and 500% elongation was also measured. The results for these findings can be found in the following tables:

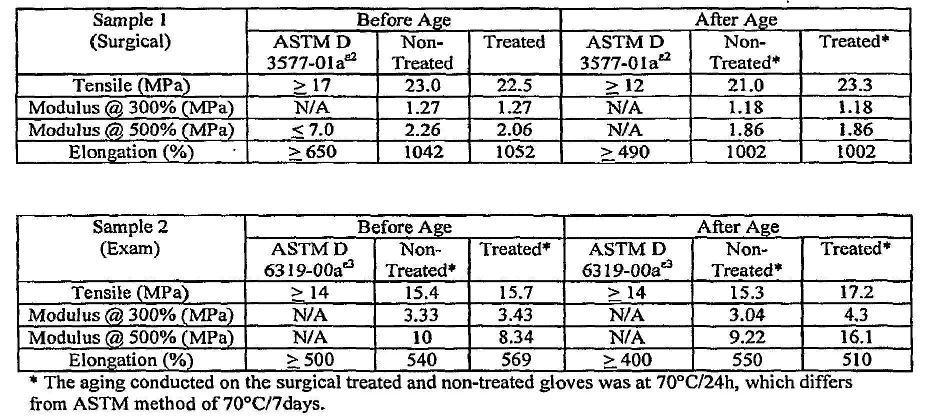

The glove tensile strength and elongation of the treated surgical and exam gloves are not only similar to non-treated gloves, but also meet the ASTM standards for surgical and exam gloves. The nonaqueous coating of the present invention does not have any negative impact on the physical properties of the treated gloves.

The coatings according to the present invention can be applied to various substrates including, but not limited to, medical gloves made with different materials such as natural rubber, polyisoprene, polyvinyl chloride, nitrile rubber, polychloroprene rubber, thermoplastic elastomers, and the like. Further, combinations of any of these materials are also possible.

### Industrial Applicability:

The invention provides a coating composition for the skin-contacting surfaces of elastomeric articles which provide beneficial therapeutic skin treatment to the wearer's skin. The advantages afforded by the invention are particularly useful in elastomeric articles which are associated with prolonged periods of wear, such as surgical gloves, where deterioration of skin quality can occur as a result of such prolonged wear. Elastomeric articles made according to the invention can be used in a variety of contexts in addition to the medical field, such as food preparation or cosmetic usage environments. Accordingly, gloves made according to the invention not only reduce the adverse effects on the wearer's skin typically associated with elastomeric gloves worn for long periods of time, but improve the condition of the wearer's skin.

## Claims

1. A nonaqueous therapeutic coating composition for the skin-contacting surface of an elastomeric article, said coating composition comprising:
glycerin in an amount ranging from 50.00% to 90.00% by weight of the composition; and
sorbitol in an amount ranging from 0.1% to 6.0% by weight of the composition;
wherein said composition is essentially water-free and transfers upon contact with skin; and,
wherein the nonaqueous therapeutic coating composition is adapted for application directly onto the skin-contacting surface of the elastomeric article.

2. The therapeutic coating composition according to claim 1, which additionally comprises a hydration promoter.

3. The therapeutic coating composition according to claim 2, wherein said hydration promoter comprises sodium citrate.

4. The therapeutic coating composition according to claim 3, which additionally comprises an organic solvent.

5. An elastomeric article comprising the therapeutic coating composition of claim 1 on the skin-contacting surface of said article.

6. A nonaqueous therapeutic coating composition for the skin-contacting surface of an elastomeric article, said coating composition comprising:
glycerin in an amount ranging from 50.00% to 90.00% by weight of the composition;
sorbitol in an amount ranging from 0.1% to 6.0% by weight of the composition; and
a transferable film-forming polymer, an exfoliant, or microporous particles;
wherein said composition is essentially water-free and transferable upon contact with skin; and,
wherein the nonaqueous therapeutic coating composition is adapted for application directly onto the skin-contacting surface of the elastomeric article.

7. The therapeutic coating composition according to claim 6, wherein said film-forming polymer is selected from the group consisting of polysaccharides, cellulose and cellulose derivatives, polyvinyl pyrrolidone and polyvinyl pyrrolidone derivatives, and combinations thereof.

8. The therapeutic coating composition according to claim 7, wherein said polysaccharide comprises chitosan.

9. An elastomeric article comprising the therapeutic coating composition of claim 6 on the skin-contacting surface of said article.

10. The therapeutic coating composition according to Claim 6, wherein said exfoliant comprises hydroxyacid.

11. The therapeutic coating composition according to claim 10, wherein said hydroxyacid comprises gluconolactone.

12. A process for the production of an elastomeric article, said process comprising contacting the skin contacting surface of said elastomeric article with a nonaqueous therapeutic coating composition according to any one of claims 1 and 6.

## Patentansprüche

1. Nichtwässrige, therapeutische Beschichtungszusammensetzung für die Hautkontaktfläche eines Elastomerartikels, wobei die Beschichtungszusammensetzung Folgendes beinhaltet
Glyzerin in einer Menge von 50,00 Gewichtsprozent bis 90,00 Gewichtsprozent der Zusammensetzung; und
Sorbitol in einer Menge von 0,1 Gewichtsprozent bis 6,0 Gewichtsprozent der Zusammensetzung;
wobei die Zusammensetzung im Wesentlichen wasserfrei ist und sich bei Kontakt mit der Haut überträgt; und,
wobei die nichtwässrige, therapeutische Beschichtungszusammensetzung zum Auftrag direkt auf die Hautkontaktfläche des Elastomerartikels geeignet ist.

2. Therapeutische Beschichtungszusammensetzung nach Anspruch 1, welche zudem einen Hydrierungsförderer beinhaltet.

3. Therapeutische Beschichtungszusammensetzung nach Anspruch 2, bei welcher der Hydrierungsförderer Natriumcitrat beinhaltet.

4. Therapeutische Beschichtungszusammensetzung nach Anspruch 3, welche zudem ein organisches Lösemittel beinhaltet.

5. Elastomerartikel, welcher die therapeutische Beschichtungszusammensetzung nach Anspruch 1 an der Hautkontaktseite des Artikels beinhaltet.

6. Nichtwässrige, therapeutische Beschichtungszusammensetzung für die Hautkontaktfläche eines Elastomerartikels, wobei die Beschichtungszusammensetzung Folgendes beinhaltet
Glyzerin in einer Menge von 50,00 Gewichtsprozent bis 90,00 Gewichtsprozent der Zusammensetzung;
Sorbitol in einer Menge von 0,1 Gewichtsprozent bis 6,0 Gewichtsprozent der Zusammensetzung; und
ein übertragbares, filmbildendes Polymer, ein Exfoliationsmittel oder mikroporöse Partikel;
wobei die Zusammensetzung im Wesentlichen wasserfrei ist und bei Kontakt mit der Haut übertragbar ist; und,
wobei die nichtwässrige, therapeutische Beschichtungszusammensetzung zum Auftrag direkt auf die Hautkontaktfläche des Elastomerartikels geeignet ist.

7. Therapeutische Beschichtungszusammensetzung nach Anspruch 6, bei welcher das filmbildende Polymer aus der Gruppe gewählt ist, bestehend aus Polysacchariden, Zellulose und Zellullosederivaten, Polyvinylpyrrolidon und Polyvinylpyrrolidonderivaten und Kombinationen hieraus.

8. Therapeutische Beschichtungszusammensetzung nach Anspruch 7, bei welcher das Polysaccharid Chitosan beinhaltet.

9. Elastomerartikel, welcher die therapeutische Beschichtungszusammensetzung nach Anspruch 6 an der Hautkontaktseite des Artikels beinhaltet.

10. Therapeutische Beschichtungszusammensetzung nach Anspruch 6, bei welcher das Exfoliationsmittel Hydroxysäure beinhaltet.

11. Therapeutische Beschichtungszusammensetzung nach Anspruch 10, bei welcher die Hydroxysäure Gluconolacton beinhaltet.

12. Verfahren zur Herstellung eines Elastomerartikels, wobei das Verfahren Inkontaktbringen der Hautkontaktfläche des Elastomerartikels mit einer nichtwässrigen, therapeutischen Beschichtungszusammensetzung nach einem der Ansprüche 1 und 6 beinhaltet.

## Revendications

1. Composition de revêtement thérapeutique non aqueuse pour la surface de contact avec la peau d'un article en élastomère, ladite composition de revêtement comprenant :
de la glycérine selon une quantité qui s'inscrit dans une plage qui va de 50,00 % à 90,00 % en poids de la composition ; et
du sorbitol selon une quantité qui s'inscrit dans une plage qui va de 0,1 % à 6,0 % en poids de la composition ; dans laquelle :
ladite composition est essentiellement exempte d'eau et elle est transférée suite à un contact avec la peau ; et dans laquelle :
la composition de revêtement thérapeutique non aqueuse est adaptée pour son application directement sur la surface de contact avec la peau de l'article en élastomère.

2. Composition de revêtement thérapeutique selon la revendication 1, laquelle comprend de façon additionnelle un promoteur d'hydratation.

3. Composition de revêtement thérapeutique selon la revendication 2, dans laquelle ledit promoteur d'hydratation comprend du citrate de sodium.

4. Composition de revêtement thérapeutique selon la revendication 3, laquelle comprend de façon additionnelle un solvant organique.

5. Article en élastomère comprenant la composition de revêtement thérapeutique selon la revendication 1 sur la surface de contact avec la peau dudit article.

6. Composition de revêtement thérapeutique non aqueuse pour la surface de contact avec la peau d'un article en élastomère, ladite composition de revêtement comprenant :
de la glycérine selon une quantité qui s'inscrit dans une plage qui va de 50,00 % à 90,00 % en poids de la composition ;
du sorbitol selon une quantité qui s'inscrit dans une plage qui va de 0,1 % à 6,0 % en poids de la composition ; et
un polymère de formation de film transférable, un exfoliant ou des particules microporeuses ; dans laquelle :
ladite composition est essentiellement exempte d'eau et elle peut être transférée suite à un contact avec la peau ; et dans laquelle :
la composition de revêtement thérapeutique non aqueuse est adaptée pour son application directement sur la surface de contact avec la peau de l'article en élastomère.

7. Composition de revêtement thérapeutique selon la revendication 6, dans laquelle ledit polymère de formation de film est sélectionné parmi le groupe qui est constitué par les polysaccharides, la cellulose et les dérivés de cellulose, le polyvinylpyrrolidone et les dérivés de polyvinylpyrrolidone ainsi que leurs combinaisons.

8. Composition de revêtement thérapeutique selon la revendication 7, dans laquelle ledit polysaccharide comprend du chitosane.

9. Article en élastomère comprenant la composition de revêtement thérapeutique selon la revendication 6 sur la surface de contact avec la peau dudit article.

10. Composition de revêtement thérapeutique selon la revendication 6, dans laquelle ledit exfoliant comprend de l'hydroxyacide.

11. Composition de revêtement thérapeutique selon la revendication 10, dans laquelle ledit hydroxyacide comprend du gluconolactone.

12. Procédé pour la fabrication d'un article en élastomère, ledit procédé comprenant la mise en contact de la surface de contact avec la peau dudit article en élastomère avec une composition de revêtement thérapeutique non aqueuse selon l'une quelconque des revendications 1 et 6.
